(19) Europäisches Patentamt
European Patent Office
Office européen des brevets

(11) **EP 2 459 065 B1**

(12) **EUROPEAN PATENT SPECIFICATION**

(45) Date of publication and mention
of the grant of the patent:
**08.01.2014 Bulletin 2014/02**

(51) Int Cl.:
***A61B 5/11*** *(2006.01)*

(21) Application number: **10742294.1**

(22) Date of filing: **23.07.2010**

(86) International application number:
**PCT/IB2010/053364**

(87) International publication number:
**WO 2011/013048 (03.02.2011 Gazette 2011/05)**

(54) **METHOD AND APPARATUS FOR THE ANALYSIS OF A BALLISTOCARDIOGRAM SIGNAL**

VERFAHREN UND VORRICHTUNG ZUR ANALYSE EINES BALLISTOKARDIOGRAMMSIGNALS

PROCEDE ET APPAREIL POUR L'ANALYSE D'UN SIGNAL DE BALLISTOCARDIOGRAMME

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **31.07.2009 EP 09166924**

(43) Date of publication of application:
**06.06.2012 Bulletin 2012/23**

(73) Proprietors:
• **Koninklijke Philips N.V.**
**5656 AE Eindhoven (NL)**
Designated Contracting States:
**AL AT BE BG CH CY CZ DK EE ES FI FR GB GR HR HU IE IS IT LI LT LU LV MC MK MT NL NO PL PT RO SE SI SK SM TR**
• **Philips Intellectual Property & Standards GmbH**
**20099 Hamburg (DE)**
Designated Contracting States:
**DE**

(72) Inventors:
• **BRUESER, Christoph**
**NL-5656 AE Eindhoven (NL)**
• **STADLTHANNER, Kurt**
**NL-5656 AE Eindhoven (NL)**
• **FRIEDRICH, David**
**NL-5656 AE Eindhoven (NL)**
• **BRAUERS, Andreas**
**NL-5656 AE Eindhoven (NL)**

(74) Representative: **Smit, Frederik Jan**
**Cordian B.V.**
**High Tech Campus 27**
**5656 AE Eindhoven (NL)**

(56) References cited:
**WO-A1-2008/095318        WO-A1-2009/073982**
**WO-A1-2010/067294        WO-A1-2010/067297**
**WO-A2-2007/143535**

• **KORTELAINEN J M ET AL: "FFT averaging of multichannel BCG signals from bed mattress sensor to improve estimation of heart beat interval" ENGINEERING IN MEDICINE AND BIOLOGY SOCIETY, 2007. EMBS 2007. 29TH ANNUAL INTERNATIONAL CONFERENCE OF THE IEEE, IEEE, PISCATAWAY, NJ, USA, 22 August 2007 (2007-08-22), pages 6685-6688, XP031337773 ISBN: 978-1-4244-0787-3**
• **JANSEN B H ET AL: "MONITORING OF THE BALLISTOCARDIOGRAM WITH THE STATIC CHARGE SENSITIVE BED" IEEE TRANSACTIONS ON BIOMEDICAL ENGINEERING, IEEE SERVICE CENTER, PISCATAWAY, NJ, US LNKD- DOI: 10.1109/10.83586, vol. 38, no. 8, 1 August 1991 (1991-08-01), pages 748-751, XP000260758 ISSN: 0018-9294**

EP 2 459 065 B1

**Description**

TECHNICAL FIELD OF THE INVENTION

The invention relates to a method and apparatus for the analysis of a ballistocardiogram signal, and in particular to a method and apparatus that provides for the detection of single heart beat events in a ballistocardiogram signal.

BACKGROUND TO THE INVENTION

A ballistocardiograph (BCG) measures the movement of the human body due to the momentum of the blood as it is pumped by the heart.

The BCG has advantages over the electrocardiograph (ECG) in that the measurement of body vital signs is possible without electrodes having to be glued to the body or for special sensors like belts, textiles or the like to be worn. Due to this unobtrusive nature the BCG is best suited for monitoring the heart activity of people at night over a long period of time. Likewise, BCG systems can be used as an additional safety measure to monitor patients in the general ward of hospitals without reducing their sleep quality. Since modem BCG systems can be fully integrated into a bed and can be activated by a single switch, the additional safety provided by such a system requires minimal extra effort from healthcare professionals.

Currently, algorithms for analysing ballistocardiogram signals to determine the heart rate use spectral methods or methods in the time domain that detect the reoccurrence of certain patterns by, for example, evaluating the autocorrelation function of the signal. In all of these approaches, segments of the signal have to be considered which last for several seconds such that they cover multiple heart beats. As a result, average heart beats over a period of time are obtained, but no beat-to-beat information is available.

Some algorithms for beat-to-beat estimation from ballistocardiogram signals have been presented, but these either require a large and expensive sensor array in order to work properly ("FFT averaging of multichannel BCG signals from bed mattress sensor to improve estimation of heart beat interval" by Kortelainen, J.M. and Virkkala, J., Engineering in Medicine and Biology Society, 2007, EMBS 2007, 29th Annual International Conference of the IEEE, 22-26 August 2007, pages 6685-6688), human interaction ("Automatic Ballistocardiogram (BCG) Beat Detection Using a Template Matching Approach" by J.H. Shin, B.H. Choi, Y.G. Lim, D.U. Joeng and K.S. Park, Engineering in Medicine and Biology Society, 2008, EMBS 2008, 30th Annual International Conference of the IEEE, 21-24 August 2008) or use different sensor modalities and lack accuracy ("Estimation of Respiratory Waveform and Heart Rate Using an Accelerometer" by D.H. Phan, S. Bonnet, R. Guillemaud, E. Castelli, N.Y. Pham Thi, Engineering in Medicine and Biology Society, 2008, EMBS 2008, 30th Annual International Conference of the IEEE, 21-24 August 2008).

It is questionable whether these algorithms can be brought to market, or whether they are able to deal with the high intra- and inter-patient variability of ballistocardiogram signals, particularly those patients with arrhythmias.

Arrhythmias are a widespread problem and can be a predictor of critical health conditions. According to the American Heart Association an estimated 2.2 million Americans are living with atrial fibrillations. This means that the assumption made by some algorithms of a regular beating heart does not hold for a significant fraction of the people to be monitored in the general wards of hospitals, for instance.

Depending on the severity of arrhythmias, existing algorithms that try to detect heart beats in BCG signals fail sooner or later since they usually assume that the heart is beating rather regularly. Thus, there is a need for an algorithm for processing ballistocardiogram signals that is not based on this regularity assumption and which is thus able to work also in the case of severe arrhythmia. It is also desirable for an algorithm that can provide a reliable beat to beat analysis of the BCG signal.

Furthermore, many of the existing BCG analysis algorithms only work offline. Thus, it is desirable for the algorithm to be suitable for use online (i.e. continuously) and therefore suitable for monitoring and alerting tasks in hospitals, nursing homes or at home.

SUMMARY OF THE INVENTION

According to a first aspect of the invention there is provided a method of detecting heart beats of a user in a ballistocardiogram, BCG, signal, the method comprising the steps according to claim 1.

According to a second aspect of the invention, there is provided an apparatus for use with a device for measuring a ballistocardiogram signal of a user, the apparatus comprising means for receiving a ballistocardiogram signal from the device; and processing means for performing the method described above on the received ballistocardiogram signal.

According to a third aspect of the invention, there is provided a computer program product comprising computer program code that, when executed on a computer or processor, is configured to cause the computer or processor to perform the method described above.

**BRIEF DESCRIPTION** OF THE DRAWINGS

The invention will now be described in the following detailed description, by way of example only, with reference to the following drawings, in which:

Fig. 1 is a flow chart illustrating the principle of detecting heart beats according to the invention;
Fig. 2 is a graph illustrating an unfiltered ballistocardiogram signal;
Fig. 3 is a flow chart illustrating a method of training the algorithm according to the invention;
Fig. 4 is a graph illustrating the result of filtering the ballistocardiogram signal to remove the breathing component;
Fig. 5 is a graph illustrating the result of smoothing the filtered ballistocardiogram signal;
Fig. 6 is an illustration of the parameters derived from the ballistocardiogram signal in accordance with an embodiment of the invention;
Fig. 7 is a flow chart illustrating the implementation of step 107 in Figure 3 in more detail;
Fig. 8 is a graph illustrating the high frequency filter response envelope of the ballistocardiogram signal;
Fig. 9 is a flow chart illustrating the method of using the algorithm to detect heart beats in a ballistocardiogram signal;
Fig. 10 is a graph illustrating reliability score triplets;
Fig. 11 is a graph illustrating the effectiveness of the algorithm in relation to heart beats detected using an ECG;
Fig. 12 is a graph comparing the beat to beat intervals determined by the algorithm and by using an ECG;
Fig. 13 is a graph illustrating the effectiveness of the algorithm in detecting a heart beat when arrhythmia is present in relation to an ECG; and
Fig. 14 is a block diagram of an apparatus in accordance with an embodiment of the invention.

DETAILED DESCRIPTION OF THE PREFERRED EMBODIMENTS

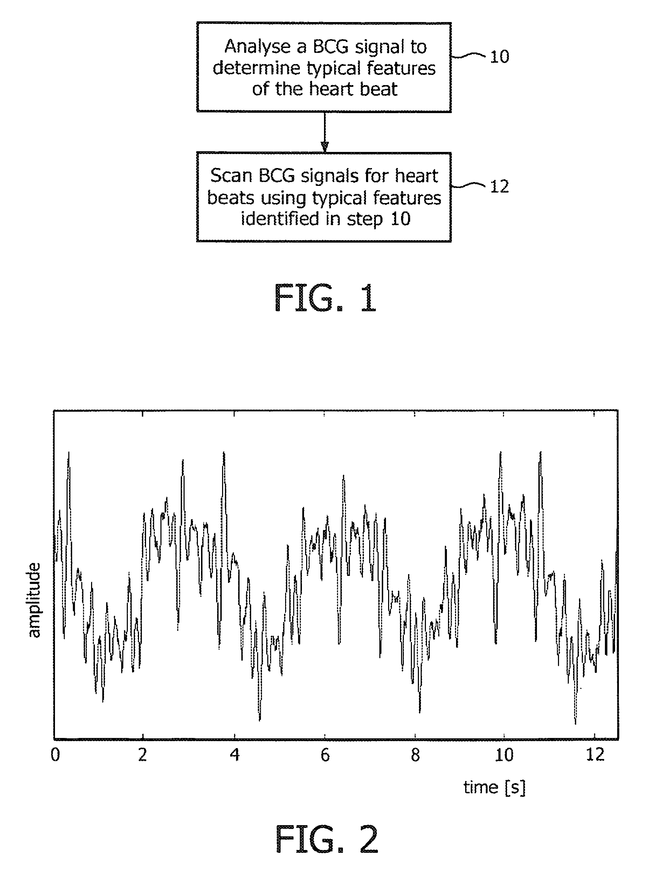

The flow chart in Figure 1 illustrates the general principles used by the algorithm according to the invention. In the first step (step 10), the training step, a short segment of a ballistocardiogram (BCG) signal is analyzed and the typical features of the heart beat are determined. In the second step (step 12), the assignment step, newly recorded samples of a live BCG signal are scanned for heart beats, or, in other words, the algorithm tries to rediscover the features learned in the training step in the newly recorded signals. The operation of the training and assignment steps are briefly outlined below.

In order to learn the typical features of the heart beat during the training period (step 10), the segment of the ballistocardiogram used for the training is first filtered in order to remove any breathing components and to smooth the signal. Specific points are identified in the resulting signal which can be used to parameterize the signal. At each specific point the next n parameters are used to form a feature vector. The resulting feature vectors are clustered by similarity. Here, the principle is that the feature vectors covering individual heart beats will be similar enough that they will be clustered together, while other feature vectors will be found in different clusters.

Alongside this processing, the original (unfiltered) training segment is filtered in order to remove the breathing signal, but this time, no smoothing occurs. In this signal, high frequency components are determined which usually coincide with each heart beat. In this process, the passband frequencies of the filter used to detect the high frequency components are adjusted automatically.

The cluster that contains the feature vectors relating to the heart beats is identified using the characteristics of the individual clusters together with the determined high frequency components.

During the assignment step (step 12) the newly measured or received signal is again filtered, smoothed and parameterized as described above. The parameters of the signal are compared with the cluster that was identified as containing the features of the heart beats in the training step (step 10). Furthermore, high frequency components are detected in the newly arriving signal. Based on the similarity to the cluster containing the heart beats and the location of the high frequency components, a first estimate of the locations of heart beats is made. The positions of heart beats given by the first estimate are calibrated in a refinement step which is based on an autocorrelation between two consecutive heart beat signals.

The operation of the algorithm according to the invention will now be described in more detail with reference to Figures 2 to 10 of the accompanying drawings.

Figure 2 shows a typical segment of a BCG signal consisting of a breathing component which expresses itself as a low frequency quasi-sinusoidal signal plus a heart beat component which leads to sharp bursts. The goal of the presented algorithm is to identify the bursts related to the heart beat and to determine the pulse-to-pulse (otherwise known as the beat-to-beat) time distance between them. For this purpose, as summarised above, the algorithm first detects and learns the characteristic features of the heart beat.

It will be appreciated that these features change from individual to individual and also depend strongly on the position of the monitored person relative to the BCG sensor.

Learning step

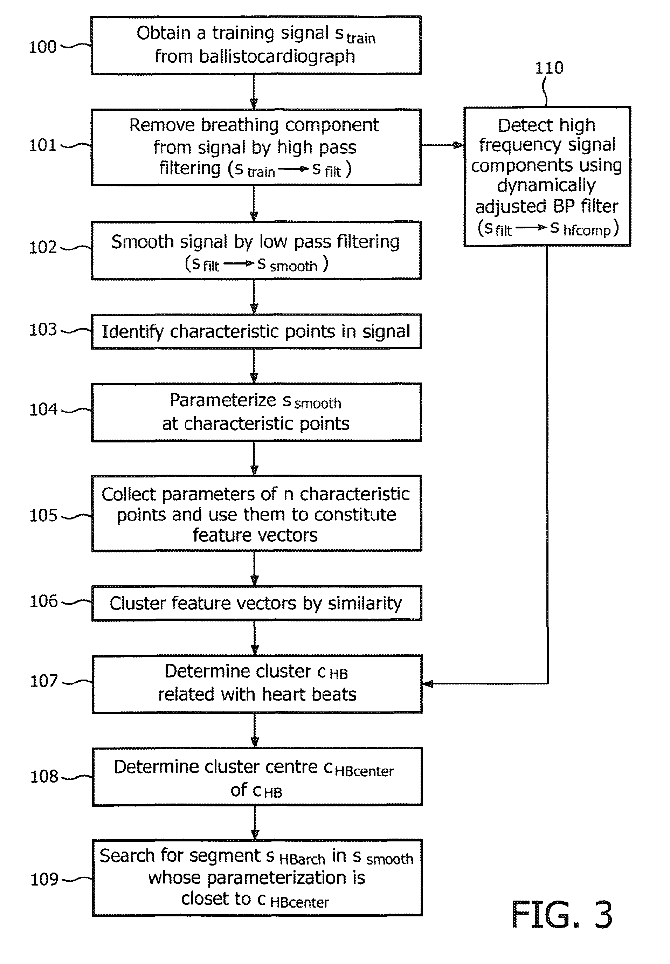

The learning step (step 10) of the algorithm is depicted in Figure 3. In step 100, a training signal, denoted $s_{train}$ comprising a segment of a ballistocardiogram signal is obtained. The training segment must be long enough such that it contains a number of heart beats, and it must be clear of artefacts. This segment is preferably between 10 and 60 seconds long.

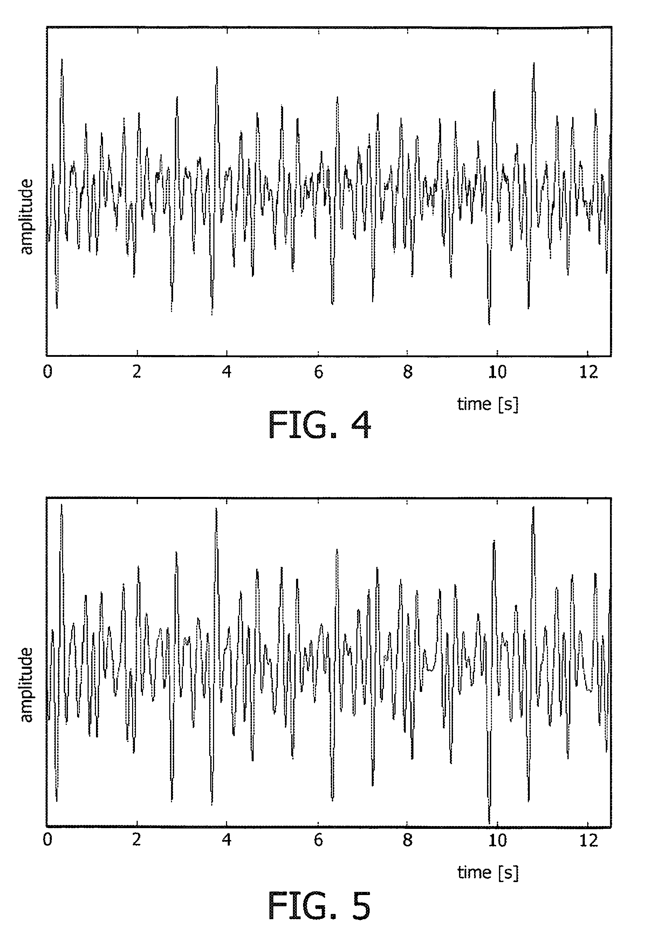

In step 101, the breathing component is removed from the ballistocardiogram signal by means of a high pass filter with a cut-off frequency of 1 Hz. The resulting filtered signal $s_{filt}$ (depicted in Figure 4) is used in two different processing paths, as described further below.

In the later steps of the algorithm every local extremum (i.e. maximum or minimum) of the pre-processed signal is analyzed. In these steps only meaningful, well-expressed extrema should be taken into account, while considering negligibly small extrema hampers the success of the algorithm. For this reason, the segment is first smoothed by means of a low pass filter with a cut-off frequency of 10 Hz (step 102 in the flowchart of Figure 3) in the first path of the algorithm (steps 102 to 106). Figure 5 shows the smoothed signal, $s_{smooth}$. As can be seen, high frequency noise and hence also small local extrema still visible in Figure 4 are removed such that only the basic shape of the signal remains.

In the BCG signal $s_{smooth}$ the heart beats are discernible as groups of peaks with similar amplitudes and inter-peak-distances after the breathing component has been removed.

In step 103, characteristic points the filtered and smoothed BCG signal are detected. In a preferred embodiment, these characteristic points are the maximas and the minimas of the signal.

In step 104 each detected local maxima in the signal $s_{smooth}$ is parameterized. In a preferred embodiment, each local maxima k is parameterised using the following features:

i) the amplitude ($a_{max}$) of the maxima,
ii) the distance ($d_{max}$) between the local maxima and the next local minima to the right,
iii) the amplitude ($a_{min}$) of the next local minima to the right of the local maxima, and
iv) the distance ($d_{min}$) between the next local minima to the right of the local maxima and the next local maxima to the right.

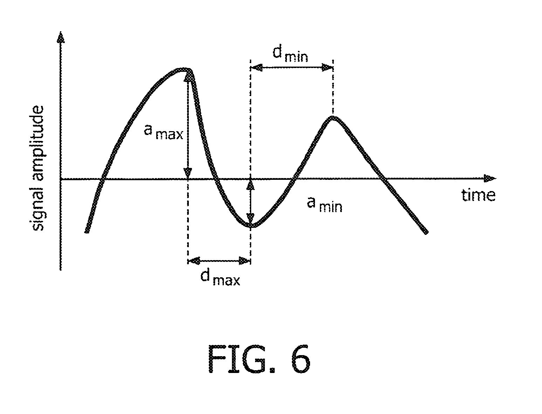

These parameters are illustrated in Figure 6 and they give a rough description of the shape of the signal.

In step 105 the parameters of n (with n usually in the range n = 3 to 10) consecutive peaks are collated and used to form feature vectors $f_k$. A feature vector $f_k$ is given by

$$f_k = [a_{max,k}, d_{max,k}, a_{min,k}, d_{min,k}, a_{max,k+1}, d_{max,k+1}, a_{min,k+1}, d_{min,k+1}, \ldots,$$

$$a_{max,k+n-1}, d_{max,k+n-1}, a_{min,k+n-1}, d_{min,k+n-1}]$$

$$(1)$$

where k = 1, 2, ..., K-n+1, and K denotes the number of peaks in the training segment $s_{train}$. The number of peaks n to be collated into each feature vector $f_k$ roughly corresponds to the number of peaks which appear during each heart beat in the BCG signal. Thus, step 105 results in K-n+1 distinct feature vectors $f_k$.

It will be appreciated by those skilled in the art that other parameterizations of the BCG signal $s_{smooth}$ are possible. For instance, the signal can be approximated by splines where the knots of the spline approximation are the local maxima and minima and possibly a low number of other points (usually only one) in-between them. Alternatively, parameterization of the signal in terms of its most significant Fourier coefficients is possible.

In step 106, the feature vectors $f_k$ are clustered by similarity. Prior to this, in some embodiments of the invention, as clustering methods usually work best in low rather than high dimensional vector spaces, the dimension of the feature vectors can be reduced by means of principal component analysis.

In the BCG signal $s_{smooth}$, the heart beats are visible as repetitive patterns of peaks. Therefore, the particular feature vectors that describe the heart beats will be similar and will be distinguished considerably from those feature vectors that describe random fluctuations of the signal in-between the beats.

Therefore, in step 106, grouping the feature vectors by similarity will lead to a cluster or clusters that contain the feature vectors related to the heart beats only.

Those skilled in the art will appreciate that various methods, like, for instance, k-means clustering, hierarchical clustering, self organizing maps and the like are suitable for grouping the feature vectors $f_k$ into clusters. It will also be appreciated that various distance measures d, like, for instance, the Euclidian distance, the angle between the individual feature vectors etc. can be used in the clustering step.

In a preferred embodiment of the invention, apart from the standard clustering methods, it is of advantage to form a cluster for each feature vector that contains, each time, the P (where P is in the region of 10) next neighbours with respect to a certain distance measure (preferably the Euclidian distance).

Any of the clustering techniques leads to a number of clusters M, and it is necessary to identify the cluster that contains the feature vectors describing the heart beat patterns (step 107).

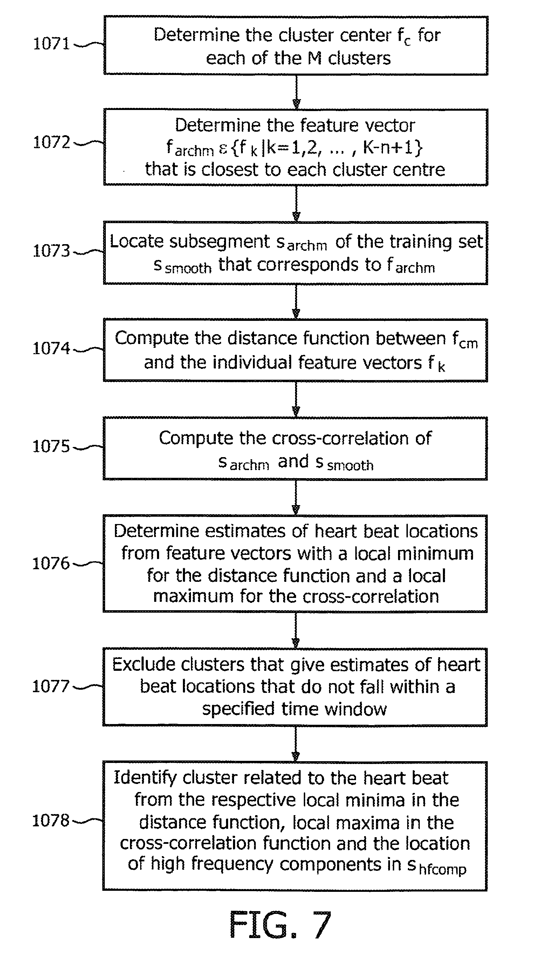

The flow chart in Figure 7 illustrates the steps carried out in step 107.

In step 1071, for each of the M clusters, the cluster centre $f_{cm}$ is determined (where m indicates the m-th cluster and m = 1, ..., M). Techniques for determining cluster centres are well known to those skilled in the art, and will not be described further herein.

In step 1072, for each of the M cluster centres identified in step 1071, the feature vector $f_k$ in the set $\{f_k| k=1,2,..., K-n+1\}$ that is closest to the cluster centre $f_{cm}$ is identified. This feature vector is denoted as $f_{archm}$, as it is the feature vector that is the archetype or model of the part of the signal represented by the m$^{th}$ cluster. Thus, for the cluster that represents the heart beat signal, the archetype feature vector $f_{arch}$ or the cluster centre $f_c$ for that cluster can be seen as a model for a parameterised heart beat.

Next, in step 1073, the training signal $s_{smooth}$ is searched for the subsegment $s_{archm}$ that best corresponds to $f_{archm}$. Effectively, steps 103 and 104 of the learning procedure are reversed for the feature vector $f_{archm}$). Thus, the located subsegment $s_{archm}$ contains those n peaks of $s_{smooth}$ whose parameterization is closest to $f_{archm}$.

Steps 1074-1078 described below indicate how the cluster and cluster centre representing the heart beat signal are identified from the M subsegments $s_{arch}$ and M cluster centres identified in step 1072. Although $f_c$ is used in the following steps, it will be appreciated that $f_{arch}$ could be used instead.

Firstly, in step 1074, the distance function between each cluster centre $f_{cm}$ and the individual feature vectors $f_k$ is computed. Preferably, the same distance measure as used in the clustering step (step 106) is used.

This distance function d will have a local minima with respect to $f_{cm}$ at position p, where $p \in [1,2,...,K-n+1]$ when the following condition is fulfilled: $d(f_c, f_{p-1}) > d(f_c, f_p) < d(f_c,f_{p+1}$

It will be appreciated that the distance function d between $f_{cm}$ and any feature vector related to or that is part of that cluster will have a local minimum.

Then, in step 1075, the cross correlation between each subseqment $s_{archm}$ and the training signal $s_{smooth}$ is determined. It will be appreciated that the results of cross correlation will have maxima where the subsegment $s_{archm}$ is most similar to the training signal $s_{smooth}$ (i.e. the parts of the signal that the feature vectors in the m$_{th}$ cluster represent).

Considering the cluster and cluster centre that represents the heart beat (which have still not been identified from the M clusters at this stage), there will be a local minimum in the distance function and a local maximum in the cross correlation at each heart beat in the BCG signal $s_{smooth}$.

Thus, in step 1076, first estimates of heart beat locations are determined separately for each of the M clusters and cluster centres using the minima of the distance function and the maxima of the cross correlation function.Further details about how these two functions are used to identify locations is given in the assignment section below.

In addition to the cluster and cluster centre relating to the heart beat signal providing locations for the heart beats, the other clusters will also identify locations in $s_{smooth}$ having local minima in the distance function and local maxima in the cross correlation function. However, these local extrema generally only appear sporadically in the BCG signal.

Hence clusters that are not related to the heart beat can be identified by checking the time between two individual local extrema in the BCG signal (step 1077). If the intervals between consecutive "heart beats" are larger than say, 3 seconds and/or smaller than 0.25 seconds then it can be assumed that the corresponding cluster is not related with the heart beat and can be ignored in the further analysis.

Although the check in step 1077 above is useful in excluding many of the clusters from the analysis, it is often the case that more than one cluster does pass the check. Hence, a further knock-out criterion is needed.

It is a general observation that heart beats lead to segments with comparatively high frequencies in the BCG. In the literature ("Higher Frequency Phenomena in the Normal Ballistocardiogram" by J. N. Edson, R. Dickes, G. H. Flamm and M. Tobin, Circulation Research, 1958, Vol. 1, pages 405-409) these high frequency components are said to be related with the mechanical forces caused by the opening and closing of the cardiac valves. Since the frequencies of these components can vary individually but also depending on the location of the BCG sensor, a dynamically adjusted bandpass filter is used to detect them in this method (see step 110 in Figure 3).

The optimal parameters are determined by filtering the signal segment $s_{filt}$ (from step 101 in Figure 3) with various bandpass filters. The set of bandpass filters is constructed so that each individual filter has a bandwidth of 2 Hz and the whole set covers the frequency range from 4 Hz up to 22 Hz in 0.1-0.5 Hz increments. Thus, the set of bandpass filters can comprise filters having cut-off frequencies = {[4 Hz, 6 Hz], [4.1 Hz, 6.1 Hz], [4.2 Hz, 6.2 Hz], ..., [20Hz, 22Hz]}).

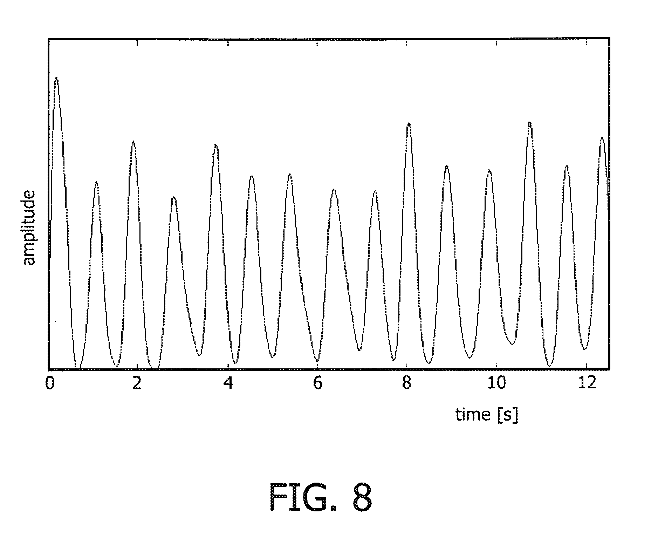

For each filter in the set, the filter response is squared and a lowpass filter with a cut-off frequency of 3.5 Hz is applied to obtain a filter response envelope (as shown in Figure 8).

For each of the filter response envelopes, a relevance score is assigned to each peak in the envelope by means of

a) the ratio between the amplitude of the peak and the average peak amplitude plus peak amplitude standard deviation, and
b) the symmetry of the peaks.

The filter which leads to the highest average relevance score is then selected and used in step 110 to detect the high frequency components of the filtered BCG signal $s_{filt}$. A more detailed description of the relevance score computation is provided in the assignment section below.

The high frequency components detected in step 110 using the optimal filter can now help to identify the cluster related to the heart beat in step 107. Returning now to Figure 7, and specifically step 1078, the cluster whose local minima in the distance function and the local maxima in the correlation function coincide best with the location of the high frequency components determined in step 110 is considered as being best related to the heart beat. Additionally, the depth of the local minima of the distance function, the amplitude of the local maxima of the cross-correlation function as well as the amplitude of the maxima of the high frequency components can be taken into account in order to determine the cluster best describing the heart beat pattern. This cluster is denoted $c_{HB}$ herein. All other clusters remaining in the analysis at this stage are now disregarded.

The cluster centre $f_c$ of this cluster is now denoted $c_{HBcentre}$ (step 108 in Figure 3) and the segment in $s_{smooth}$ that most closely matches $c_{HBcentre}$ is identified. This segment is denoted $s_{HBarch}$ (step 109). This terminates the learning part of the algorithm.

Assignment step

Once the centre $c_{HBcentre}$ and the corresponding archetype of a heart beat signal $s_{HBarch}$ have been determined, newly incoming BCG signals (in the online case) or the rest of the BCG signal (in the offline case) can be scanned for heart beats (step 12 in Figure 1). In the following, the online case will be described. Those skilled in the art will appreciate that analogous considerations also hold for the offline case.

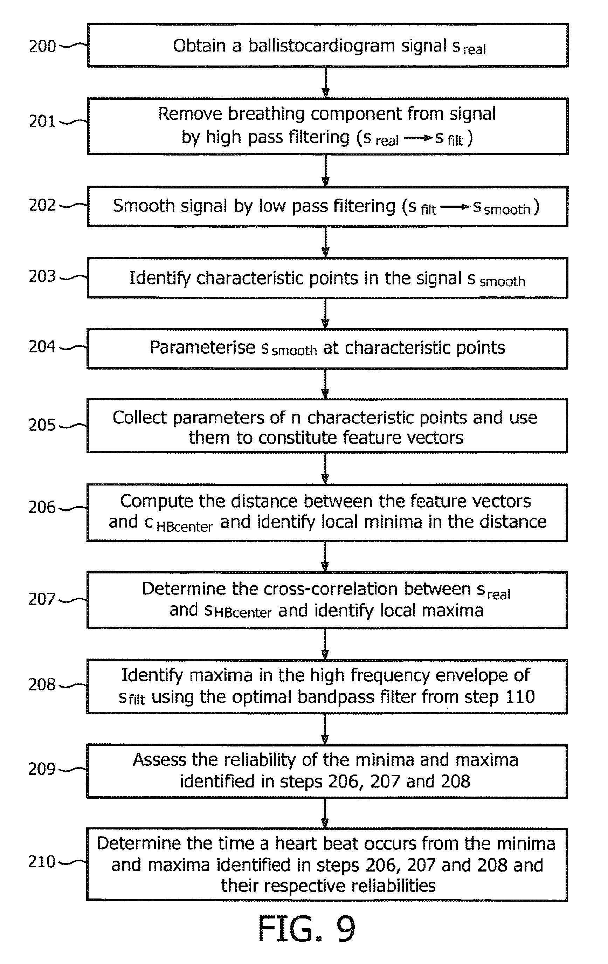

Figure 9 illustrates the method used during the assignment step.

Whenever new samples ($s_{real}$) come in from the BCG sensor they are filtered (step 201) and smoothed (step 202) as in steps 101 and 102 of the training algorithm shown in Figure 3. Likewise , characteristic points in the filtered and smoothed signal (i.e. local maxima and minima) are determined (step 203, which is the analogue of step 103). The signal is then parameterized at the characteristic points and feature vectors are constituted (steps 204 and 205).

The distances between the feature vectors and the heart beat cluster centre $c_{HBcentre}$ are computed and scanned for local minima in step 206 (which is analogous to step 1074 in Figure 7). Likewise, the cross correlation and its maxima between $s_{HBarch}$ and the newly incoming signals (after filtering and smoothing) are determined (step 207).

Further the optimal bandpass filter determined in step 110 of the learning algorithm is applied to scan the new BCG signal ($s_{filt}$) for maxima in the high frequency envelope (step 208).

The results of the analysis in steps 206, 207 and 208 can be used to identify where heart beats occur in the BCG signal $s_{real}$. However, it will be noted that the location of the minimum of the distance function, the maximum of the cross correlation and the maximum of the high frequency components might appear at slightly different locations (times). Additionally spurious high frequency components, but also wrong local extrema of the distance and cross correlation functions can appear. In such cases it is important to assess the reliability of the individual criteria in order to find a reasonable compromise between them. Thus, in step 209, the reliability of the minima and maxima found in steps 206, 207 and 208 is assessed.

The reliability of a local minimum of the distance function is evaluated by means of its depth relative to the height of the two neighbouring maxima. For this purpose the differences in amplitude between the left maxima and the minima ($h_l$) and between the right maxima and the minima ($h_r$) are computed. The reliability $r_d$ of a minimum with amplitude $a_{min}$ is then evaluated as

$$r_d = (h_l + h_r) / (2(a_{min} + \max(h_r, h_l)))$$

It can be shown that $r_d$ is normalized (i.e. $0 \le r_d \le 1$) by construction. The maximal score of 1 is assigned to minima which are symmetric with respect to the height of their neighbouring peaks and have an amplitude of 0.

Likewise a cross correlation peak with amplitude $a_{max}$ can be assessed using

$$r_x = (h_l + h_r) / (a_{max})$$

where $h_l$ and $h_r$ are the amplitude differences between the peak and the left and right minima respectively. As with $r_d$, $r_x$ is normalized and the maximal score is assigned to peaks that are symmetric with respect to their height above the left and right minima.

Finally, the reliability of a peak in the high frequency components of the signal is evaluated using

$$r_{hf} = (h_l + h_r) / (2a_{max}) \bullet (a_{max}/a_{ref})$$

In this case, the first factor of the formula is identical to the calculation of $r_x$ and thus quantifies the peak's relative symmetry, whereas the second factor adjusts the score by the ratio of the peak amplitude $a_{max}$ to a reference amplitude $a_{ref}$. The reference amplitude $a_{ref}$ is calculated during the training sequence as the average amplitude of the high frequency peaks plus the amplitudes' standard deviation.

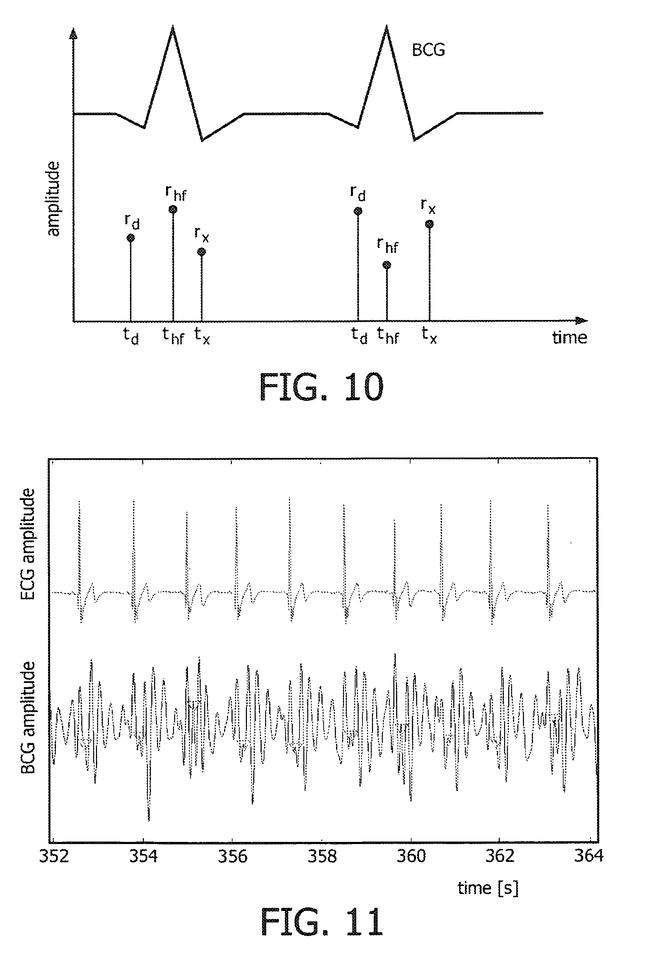

Eventually it has to be determined how the information about the reliabilities $r_x$, $r_d$, and $r_{hf}$ computed above can be combined advantageously in order to determine at what point in time a heart beat has actually occurred (step 210). The time points at which the cross correlation criterion, the distance criterion and the high frequency component criterion would detect, each by itself, a heart beat are denoted by $t_x$, $t_d$, and $t_{hf}$ respectively. In the ideal case, these three times would be identical (i.e. $t_x = t_d = t_{hf}$). However, in real world settings they slightly differ from each other because they try to detect heart beats by focusing on different features of the BCG signal. If a heart beat is well detected by all three criteria (distance, cross-correlation and high frequency component) simultaneously, the times $t_d$, $t_x$ and $t_{hf}$ will form clearly discernable triplets (see Figure 10). The aim of the following procedure is to identify these triplets and to replace them by a representative value which can be used to identify the individual heart beats.

Consider, without loss of generality, the time $t_{hf}$ as a starting point for the further description of the algorithm. At this time $t_{hf}$ the high frequency criterion suggests a heart beat. In that case the algorithm detects if $t_x$ and $t_d$ lie in the interval $[t_{hf}-c, t_{ht}+c]$ where c is a constant usually set to about 0.3 seconds. If $t_x$ and $t_d$ lie in this interval a representative value $R_{ht}(r^*_{hf}, t^*_{hf})$ of the three times $t_x$, $t_d$, and $t_{hf}$ and of the three reliabilities $r_x$, $r_d$, and $r_{hf}$ is constructed which is defined by a point in time $t^*_{hf}$ and by a reliability $r^*_{hf}$. The representative time $t^*_{hf}$ is computed as the sum of the times $t_x$, $t_d$, and $t_{hf}$ weighted with the corresponding reliabilities $r_x$, $r_d$, and $r_{hf}$ as follows:

$$t^*_{hf} = (r_x t_x + r_d t_d + r_{hf} t_{hf})/(r_x + r_d + r_{hf})$$

The representative reliability is simply computed as the sum of the reliabilities of the cross correlation, distance and high frequency criterion, i.e.

$$r^*_{hf} = (r_x + r_d + r_{hf})/N \qquad N = 3$$

Until now it has been assumed that the distance and cross correlation criterion lead to only one time $t_d$ and $t_x$, respectively, in the time interval $[t_{hf}-c, t_{hf}+c]$. If only one or even neither of these times are found in this interval, the procedure still works as described above, however the reliabilities of the missing time points are set to zero in the above formulae. Accordingly, the value of N is set to the number of non-zero reliabilities.

Likewise, it is also possible that the distance and/or correlation criterion suggest more than one heart beat in the interval $[t_{hf}-c, t_{hf}+c]$. If it is assumed that the distance criterion indicates two heart beats at times $t_{d1}$ and $t_{d2}$ in the interval $[t_{hf}-c, t_{hf}+c]$, then the corresponding reliabilities $r_{d1}$ and $r_{d2}$ are weighted by a symmetric window function centred at $t_{hf}$ which decreases linearly or nonlinearly with the distance from $t_{hf}$. In a preferred embodiment, a Gaussian window function is used which decreases to (almost) zero at the ends of the interval $[t_{hf}-c, t_{hf}+c]$. Only the time point $t_{d1}$ or $t_{d2}$ which has the largest weighted reliability is considered in the following steps of the algorithm while the other one is discarded.

So far, the procedure described above was based on the time point $t_{hf}$ as a starting point. This starting point was chosen arbitrarily, and preferably in practice the procedures outlined above are repeated with $t_d$ and $t_x$, respectively, as the starting points. This will lead to new representatives $R_d(t^*_d, r^*_d)$ and $R_x(t^*_x, r^*_x)$. In the case of clearly discernable triplets as shown in Figure 10, the time components of the representatives $t^*_d$, $t^*_x$ and $t^*_{hf}$ are identical.

For each time point t at which a heart beat is thought to have occurred, the algorithm computes a score. If no representative is found at time t (i.e. no representative has a time component $t^*_d$, $t^*_x$, or $t^*_{hf}$ that equals t) the score is zero. Otherwise, the score equals the sum of the reliability components of those representatives that have a time component equalling t.

For instance, assume that there are three representatives $R_d(t^*_d, r^*_d)$, $R_x(t^*_x, r^*_x)$ and $R_{ht}(t^*_{hf}, r^*_{hf})$ with $t^*_d = t^*_x = t^*_{hf} = t$ then the corresponding score $S(t)$ at time t equals

$$S(t) = r^*_d + r^*_x + r^*_{hf}$$

If $S(t)$ is larger than a predefined threshold $S_{thresh}$ then the algorithm detects a heart beat at time t. Otherwise no heart beat is detected and the algorithm waits for the next samples (in the online case). Those skilled in the art will appreciate that the threshold $S_{thresh}$ can be adjusted during the training and/or assignment step depending on the reliability values of the q last detected heart beats.

Furthermore, in cases where the monitored person shows a rather regular heart beat, the recently determined beat-to-beat intervals can be used to predict when the next heart beat is most likely to occur. Around these points in time the threshold $S_{thresh}$ is lowered in order to reduce the number of missed beats.

The points identified above already indicate well where the individual heart beats occurred in the BCG signal. However, in order to determine the individual beat-to-beat time intervals even more precisely, a further refinement step is required. In this step the period of time over which the pattern of a peak repeats itself for the first time in the signal is fine-tuned.

In particular, let $t_1$, $t_2$, ..., $t_N$, where N is the number of heart beats found so far, be the time points at which heart beats have been detected. Further, let $s(t_p, t_{p+1})$ denote the segment of the signal $s_{filt}$ recorded between the time points tp and $t_{p+1}$, with $1 \le p \le N-1$.

In order to fine-tune when the p-th peak repeats itself in the signal, the argument $t_{shift}$

$$t_{shift} = \arg\max_{dt} xcorr(s(t_p, t_{p+1}), s(t_{p+1} + dt, 2t_{p+1} - t_p + dt)), dt \in [-0.15 \text{ s}, 0.15 \text{ s}]$$

which maximizes the cross correlation xcorr between the segment $s(t_p, t_{p+1})$ and the following segment $s(t_{p+1}+dt, 2t_{p+1} - t_p - 1+dt)$ is determined. The beat-to-beat interval $i_{p,p+1}$ between the p-th and the p+1-th peak can eventually be computed as

$$i_{p,p+1} = t_{p+1} - t_p + dt.$$

After this step the detection of the heart beat and the beat to beat interval computation is finished and the algorithm waits for the next samples to arrive. Results

Figure 11 is a graph showing the results obtained by the algorithm according to the invention when it is applied to a short segment of BCG data where the heart beats appear rather regularly, i.e. when no arrhythmia are present. As a reference, an ECG signal obtained at the same time as the BCG signal is plotted. By comparing the two plots, it can be seen that the algorithm clearly identifies the individual heart beats in the BCG signal.

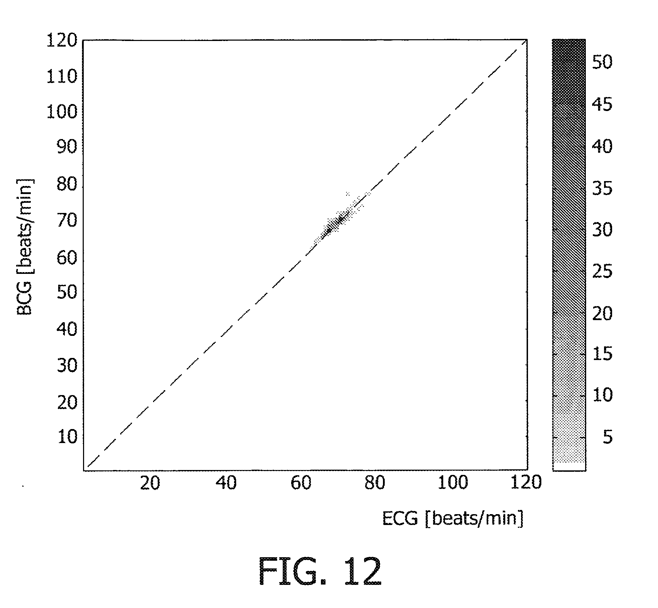

Further Figure 12 illustrates the analysis of a BCG signal and an ECG signal that were recorded simultaneously for about 8 minutes. For each new beat the time distance to the previous beat is determined in the ECG. Likewise, the algorithm according to the invention is used to compute the corresponding beat-to-beat distances based on the BCG signal. The corresponding beat-to-beat distances of both the ECG and the BCG analysis are paired together and plotted in the scatter plot shown in Figure 12. If both the BCG and the ECG analysis lead to identical estimates, the plot in Figure 12 should only contain entries along the x-y diagonal (which is indicated by a dashed line). This diagonal clearly dominates Figure 12 and only very small number of deviating pairs can be found. Averaged over the entire segment, the absolute deviation between the ECG based peak-to-peak intervals and the BCG intervals was 7 ms. Altogether only 0.6 % of all peaks were missed and 0.2 % false positives were detected.

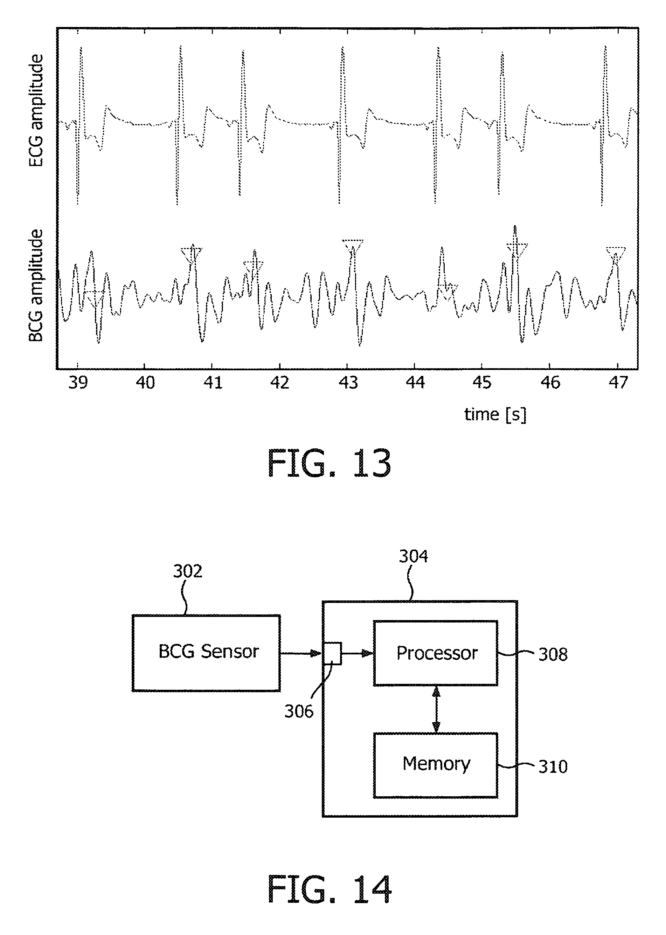

Finally, Figure 13 shows both the ECG signal and the corresponding BCG signal of an arrythmically beating heart. As in the case of the regularly beating heart (Figure 11) the algorithm according to the invention detects all heart beats in this segment. This is not possible with algorithms that are based on the assumption that the heart is beating regularly.

It will be appreciated by those skilled in the art that state of the art BCG sensors can be integrated invisibly into the bed of a person to be monitored and hence offer fully unobtrusive monitoring of the heart rate and also the breathing rate. Furthermore, BCG based monitoring systems are far cheaper than established intensive care unit (ICU) monitoring systems. These two features make BCG based solutions ideal for the general ward of hospitals where the patients are no longer in need of a full scale ICU monitoring system but in which the physicians are still interested if the vital functions of their patients. Especially in this setting the accurate detection of arrhythmia is important since they can often be an indicator or predictor for other severe health problems.

BCG based technologies can also be used at home for long term monitoring of the heart and breathing rate. This application is, for instance, of great interest for patients suffering from heart failure since in this case reduced heart rate variability is seen as a predictor of decompensations.

Further, especially due to its unobtrusive nature, the BCG can be used to assess sleep quality without disturbing the monitored person at night.

Although the invention has been described in terms of a method or algorithm, it will be appreciated that the invention can be implemented in a BCG system (i.e. a computer apparatus in combination with apparatus for measuring the BCG signals), or as a stand-alone computer system or program. It will be appreciated that the BCG system can provide a ballistocardiogram signal in analog or digital form to the inventive apparatus, and the inventive apparatus can be adapted to receive this signal accordingly. For example, the BCG system can provide the ballistocardiogram signal to the apparatus in analog form, and the apparatus can comprise an anti-alias filter and an analog-to-digital convertor for providing a digital representation of the ballistocardiogram signal to a suitably-programmed digital signal processor in the apparatus. Alternatively, the BCG system can implement an analog-to-digital convertor so the ballistocardiogram signal is provided to the apparatus (and specifically to a digital signal processor in the apparatus) in digital form. The apparatus can receive the ballistocardiogram signal using any appropriate means, such as through a wired or wireless connection to the BCG system.

One embodiment of an apparatus for implementing the invention is shown in Figure 14. A ballistocardiogram signal is provided from a BCG sensor 302 to the apparatus 304. The apparatus 304 receives the BCG signal at an input port 306 and processes the BCG signal as described in the preceding description using a processor 308. Instructions for causing the processor 308 to carry out the method can be stored in a memory 310.

There is therefore provided an improved method and apparatus for detecting single heart beat events in a ballistocardiogram signal.

While the invention has been illustrated and described in detail in the drawings and foregoing description, such illustration and description are to be considered illustrative or exemplary and not restrictive; the invention is not limited to the disclosed embodiments.

Variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality.

A single processor or other unit may fulfil the functions of several items recited in the claims. The mere fact that certain measures are recited in mutually different dependent claims does not indicate that a combination of these measured cannot be used to advantage. Any reference signs in the claims should not be construed as limiting the scope. A computer program may be stored/distributed on a suitable medium, such as an optical storage medium or a solid-state medium supplied together with or as part of other hardware, but may also be distributed in other forms, such as via the Internet or other wired or wireless telecommunication systems.

**Claims**

1. A method of detecting heart beats of a user in a ballistocardiogram (BCG) signal, the method comprising:

   detecting heart beats in the BCG signal by locating typical features of a heart beat for the user in the BCG signal (12), the typical features of the heart beat having been obtained during a training step (10), **characterized in that** the typical features of the heart beat comprise a model feature vector for a typical heart beat, $c_{HBcentre}$, and wherein the step of detecting heart beats comprises:
   identifying characteristic points in the BCG signal (203);
   determining parameters of the BCG signal at each of the identified characteristic points (204);
   constituting a plurality of feature vectors from the determined parameters (205); and
   using the plurality of feature vectors and $c_{HBcentre}$ to detect heart beats in the BCG signal (206, 207, 208, 209, 210).

2. A method as claimed in claim 1, wherein the typical features of the heart beat further comprise a portion of a BCG signal used in the training step (10) corresponding to the model feature vector, $s_{HBarch}$, and wherein the step of using comprises:

   using the plurality of feature vectors, $c_{HBcentre}$ and $s_{HBarch}$ to detect heart beats in the BCG signal (206, 207, 208, 209, 210).

3.  A method as claimed in claim 2, wherein the step of using comprises:

    computing the distance between each of the plurality of feature vectors and $c_{HBcentre}$ (206);
    identifying local minima in the resulting distances(206);
    determining the cross correlation between $s_{HBarch}$ and the BCG signal (207);
    identifying local maxima in the cross correlation(207); and
    identifying heart beats in the BCG signal from the locations of the local minima in the distances and the local maxima in the cross correlation (208, 209, 210).

4.  A method as claimed in claim 3, wherein the step of detecting heart beats (12) further comprises:

    filtering the BCG signal to obtain a high frequency component envelope (208); and
    identifying maxima in the high frequency component envelope (208);
    and wherein the step of identifying heart beats in the BCG signal comprises:

    identifying heart beats in the BCG signal from the locations of the local minima in the distances, the local maxima in the cross correlation and the local maxima in the high frequency component envelope (209, 210).

5.  A method as claimed in claim 4, wherein the step of identifying heart beats in the BCG signal further comprises:

    assessing the reliability of each of the local minima in the distances, the local maxima in the cross correlation and the local maxima in the high frequency component envelope (209).

6.  A method as claimed in claim 5, wherein the step of identifying heart beats in the BCG signal further comprises:

    forming triplets from the local minima in the distances, the local maxima in the cross correlation and the local maxima in the high frequency component envelope (210);
    determining a representative value for each triplet from the assessed reliability, the representative value indicating the time at which a heart beat occurred in the BCG signal (210).

7.  A method as claimed in any preceding claim, the method further comprising a training step (10) in which typical features of the heart beat are obtained from a portion of a BCG signal, $s_{train}$, the training step comprising:

    identifying characteristic points in $s_{train}$ (103);
    determining parameters of $s_{train}$ at each of the identified characteristic points (104);
    constituting a plurality of feature vectors from the determined parameters (105);
    grouping the plurality of feature vectors into a plurality of clusters according to the similarity of the feature vectors (106),
    identifying the cluster in the plurality of clusters related to the heart beats (107); and
    obtaining typical features of the heart beat by determining a model feature vector for a typical heart beat, $c_{HBcentre}$, from the feature vectors in the cluster and by determining a portion of a BCG signal, $s_{HBarch}$, that corresponds to the model feature vector (108, 109).

8.  A method as claimed in claim 7, wherein the step of identifying the cluster in the plurality of clusters related to the heart beats (107) comprises:

    for each cluster in the plurality of clusters:

    determining a cluster centre $f_{cm}$ (1071),
    identifying the feature vector $f_{archm}$ that most closely matches the cluster centre $f_{cm}$ (1072);
    locating the portion $s_{archm}$ of the BCG signal $s_{train}$ that corresponds to the feature vector $f_{archm}$ (1073);
    computing the distance function between the cluster centre $f_{cm}$ and each feature vector in the cluster (1074);

    computing the cross correlation of $s_{archm}$ with the BCG signal (1075); and
    determining estimates of heart beat locations in the BCG signal from feature vectors in the cluster with a local minimum for the distance function and a local maximum for the cross-correlation (1076);

filtering the BCG signal $s_{train}$ to identify locations of high frequency components (110); and
identifying the cluster related to the heart beat as the cluster that has:

(i) estimates of heart beat locations that are spaced by amounts that fall within a specified time window (1077); and
(ii) the local minima in the distance function and local maxima in the cross correlation that coincide best with the location of the high frequency components (1078).

**9.** A method as claimed in any of claims 1 to 8, wherein the characteristic points in the BCG signal comprise the maxima in the BCG signal.

**10.** A method as claimed in claim 9, wherein the parameters of each maxima point in the BCG signal comprise:

i) the amplitude ($a_{max}$) of the maxima,
ii) the distance ($d_{max}$) between the local maxima and the next local minima to the right,
iii) the amplitude ($a_{min}$) of the next local minima to the right of the local maxima, and
iv) the distance ($d_{min}$) between the next local minima to the right of the local maxima and the next local maxima to the right.

**11.** A method as claimed in any of claims 1 to 10, further comprising the step of:

reducing the dimension of the feature vectors using principal component analysis.

**12.** A method as claimed in any preceding claim, further comprising the step of:

refining the locations of heart beats detected in the BCG signal, N heart beats having been detected at times $t_1, t_2, ..., t_N$ respectively, by identifying the value of a parameter dt that maximises the cross correlation between a segment of the BCG signal between two detected heart beats at times tp and $t_{p+1}$ respectively, where $1 \leq p \leq N-1$, and a later segment of the BCG signal between time ($t_{p+1}$ + dt) and time ($2t_{p+1}$ - $t_p$ + dt).

**13.** An apparatus (304) for use with a device (302) configured to measure a ballistocardiogram signal of a user, the apparatus comprising:

means (306) configured to receive a ballistocardiogram signal from the device; and
processing means (308) configured to perform the method defined in any one of claims 1 to 12 on the received ballistocardiogram signal.

**14.** A computer program product comprising computer program code that, when executed on a computer or processor, is configured to cause the computer or processor to perform the method defined in any of claims 1 to 12.

**Patentansprüche**

**1.** Verfahren zur Erfassung von Herzschläge eines Benutzers in einem Ballistokardiogramm (BCG), wobei das Verfahren umfasst:

erfassen von Herzschläge in dem BCG-Signal durch Lokalisierung von typischen Merkmalen eines Herzschlages für den Benutzer in dem BCG-Signal (12), die typischen Merkmale des Herzschlages sind erhalten während einer Trainingsphase (10), **dadurch gekennzeichnet dass** die typischen Merkmale des Herzschlags einen Modellmerkmalvektor für einen typischen Herzschlag aufweist, $C_{HBcentre}$, und wobei die Phase des Erfassens von Herzschläge umfasst:

- identifizieren charakteristischer Punkte in dem BCG-Signal (203);
- bestimmen von Parametern des BCG-Signals an jeden der identifizierten charakteristischen Punkte (204);
- bilden einer Vielzahl von Merkmalsvektoren aus den ermittelten Parametern (205), und
- Verwendung der Vielzahl von Merkmalsvektoren und $C_{HBcentre}$ um Herzschläge in dem BCG-Signal (206, 207, 208, 209, 210) zu erfassen.

2. Verfahren nach Anspruch 1, wobei die typischen Merkmale des Herzschlags des Weiteren umfassen einen Abschnitt eines BCG-Signal gebraucht in der Trainingsphase (10) entsprechend dem Modelmerkmalsvektor, $S_{HBarch}$, und wobei der Schritt des Verwendens umfassend:

- Verwendung der Vielzahl der Merkmalsvektoren, $C_{HBcentre}$ und $S_{HBarch}$, um Herzschlägen in dem BCG-Signal (206, 207, 208, 209, 210) zu erfassen.

3. Verfahren nach Anspruch 2, wobei der Schritt des Verwendens umfassend:

- Berechnung der Abstände zwischen jedem der Vielzahl von Merkmalsvektoren und $C_{HBcentre}$ (206);
- identifizieren lokaler Minima in den erhaltenen Abständen (206); bestimmen der Kreuzkorrelation zwischen $S_{HBarch}$ und dem BCG-Signal (207);
- identifizieren lokaler Maxima der Kreuzkorrelation (207); und
- identifizieren von Herzschlägen in dem BCG-Signal aus den Positionen der lokalen Minima in den Abständen und der lokalen Maxima der Kreuzkorrelation (208, 209, 210).

4. Verfahren nach Anspruch 3, wobei die Phase des Erfassens der Herzschläge (12) ferner umfasst:

- filtern des BCG Signals um eine Hochfrequenzkomponentenumhüllende (208) zu erhalten;
- identifizieren von Maxima in der Hochfrequenzkomponentenumhüllenden (208);
und wobei der Schritt des Identifizierens von Herzschlägen in dem BCG-Signal umfasst:
- identifizieren von Herzschlägen in dem BCG-Signal aus den Positionen der lokalen Minima in den Abständen, der lokalen Maxima der Kreuzkorrelation und der lokalen Maxima der Hochfrequenzkomponentenumhüllenden (209, 210).

5. Verfahren nach Anspruch 4, wobei der Schritt des Identifizierens von Herzschlägen in dem BCG-Signal ferner umfasst:

- Beurteilung der Zuverlässigkeit von jedem der lokalen Minima in den Abständen, der lokalen Maxima der Kreuzkorrelation und der lokalen Maxima der Hochfrequenzkomponentenumhüllenden (209).

6. Verfahren nach Anspruch 5, wobei der Schritt des Identifizierens von Herzschlägen in dem BCG-Signal ferner umfasst:

- bilden von Dreiergruppen aus den lokalen Minima in den Abständen, den lokale Maxima der Kreuzkorrelation und der lokalen Maxima der Hochfrequenzkomponentenumhüllenden (210);
- ermitteln eines repräsentativen Werts für jede Dreiergruppe aus der beurteilten Zuverlässigkeit, der repräsentative Wert angebend die Zeit zu der ein Herzschlag in dem BCG-Signal (210) aufgetreten ist.

7. Verfahren nach einem der vorhergehenden Ansprüche, das Verfahren ferner umfassend eine Trainingsphase (10), in der typische Merkmale des Herzschlages aus einem Abschnitt eines BCG-Signals, $s_{train}$, erlangt sind, die Trainingsphase umfassend:

- identifizieren charakteristischer Punkte in $S_{train}$ (103);
- bestimmen von Parametern von $S_{train}$ an jedem der identifizierten charakteristischen Punkte (104);
- bilden eine Vielzahl von Merkmalsvektoren aus den ermittelten Parametern (105);
- gruppieren der Vielzahl von Merkmalvektoren in eine Vielzahl von Clustern gemäß der Ähnlichkeit der Merkmalsvektoren (106);
- identifizieren der Cluster in der Vielzahl von Clustern zugehörig zu den Herzschlägen (107), und
- erhalten von typischen Merkmalen des Herzschlags durch die Bestimmung eines Modellmerkmalsvektors für einen typischen Herzschlag, $C_{HBcentre}$, aus den Merkmalsvektoren in dem Cluster und durch die Bestimmung eines Abschnitts eines BCG-Signals, $S_{HBarch}$, der dem Modellmerkmalsvektor (108, 109) entspricht.

8. Verfahren nach Anspruch 7, wobei der Schritt des Identifizierens des Clusters in der Vielzahl von Clustern zugehörig zu dem Herzschläge (107) umfasst:

für jedes Cluster aus der Vielzahl von Clustern:

- bestimmen eines Clusterzentrum $F_{CM}$ (1071);
- Identifizierung des Merkmalsvektors $f_{archm}$, der am ehesten den Cluster Zentrum $F_{CM}$ (1072) gleicht;
- Auffinden des Abschnittes $s_{archm}$ des BCG-Signals $S_{train}$, der dem Merkmalsvektor $f_{archm}$ (1073) entspricht;
- berechnen des Abstandsfunktion zwischen dem Clusterzentrum $F_{CM}$ und jedem Merkmalsvektor in dem Cluster (1074);
- brechnen der Kreuzkorrelation der $s_{archm}$ mit dem BCG-Signal (1075); und
- bestimmen von Schätzungen der Herzschlagpositionen in dem BCG-Signal aus Merkmalsvektoren in dem Cluster mit einem lokalen Minimum für die Abstandsfunktion und einem lokalen Maximum der Kreuzkorrelation (1076);
- filtern des BCG -Signals $S_{train}$ um Positionen von Hochfrequenzkomponenten (110) zu identifizieren, und
- Identifizierung des Clusters zugehörig zum Herzschlag als das Cluster das aufweist:

> (i) Schätzungen der Herzschlagpositionen, welche angeordnet sind nach Beträge, die innerhalb eines bestimmten Zeitfensters (1077) fallen, und
>
> (ii) die lokalen Minima in der Abstandsfunktion und lokalen Maxima der Kreuzkorrelation, die am besten mit der Position der Hochfrequenzkomponenten (1078) übereinstimmen.

9. Verfahren nach einem der Ansprüche 1 bis 8, wobei die charakteristischen Punkte in dem BCG-Signal die Maxima in dem BCG-Signal umfassen.

10. Verfahren nach Anspruch 9, wobei die Parameter der einzelnen Maxima in dem BCG-Signal umfassen:

> i) die Amplitude ($a_{max}$) der Maxima,
> ii) der Abstand ($d_{max}$) zwischen dem lokalen Maximum und dem nächsten lokalen Minimum zur rechten Seite,
> iii) die Amplitude ($a_{min}$) des nächsten lokalen Minimum auf der rechten Seite des lokalen Maximums, und
> iv) der Abstand ($d_{min}$) zwischen dem nächste lokalen Minimum auf der rechten Seite des lokalen Maximums und dem nächsten lokalen Maximum auf der rechten Seite.

11. Verfahren nach einem der Ansprüche 1 bis 10, ferner umfassend den Schritt:

> Verringerung der Dimension der Merkmalsvektoren mit Verwendung der Hauptkomponentenanalyse.

12. Verfahren nach einem der vorhergehenden Ansprüche, ferner den Schritt umfassend: Verfeinern der Positionen von in dem BCG-Signal erfassten Herzschlägen, N Herzschläge entsprechend an den Zeitpunkten $t_1$, $t_2$, ..., $t_N$ sein erfasst worden, durch Identifizierung des Wertes eines Parameters dt, der die Kreuzkorrelation zwischen einem Segment des BCG-Signal zwischen zwei erfassten Herzschläge zu den entsprechenden Zeiten $t_p$ und $t_{p+1}$, mit $1 \leq p \leq N-1$, und einem späteren Segment des BCG-Signal zwischen dem Zeitpunkt ($t_{p+1}$ + dt) und dem Zeitpunkt ($2t_{p+1}$ - tp + dt) maximiert.

13. Vorrichtung (304) zur Verwendung mit einer Anlage (302) angepasst zum Messen eines Ballistokardiogrammsignals von einem Benutzer, die Vorrichtung umfassend:

> - Mittel (306) angepasst zum Empfangen eines Ballistokardiogrammsignals von der Anlage;
> - und Verarbeitungsmittel (308) angepasst zum Durchführen des Verfahrens nach einem der Ansprüche 1 bis 12 auf dem empfangenen Ballistokardiogrammsignal.

14. Ein Computerprogrammprodukt umfassend Computerprogrammcode, der wenn auf einem Computer oder einem Prozessor ausgeführt, angepasst ist den Computer oder Prozessor zu veranlassen das Verfahren nach einem der Ansprüche 1 bis 12 durchzuführen.

## Revendications

1. Une méthode de détection de battements de coeur d'un utilisateur dans un signal de ballistocardiogramme (BCG), la méthode comprenant:

> la détection des battements cardiaques dans le signal BCG en localisant des caractéristiques typiques d'un battement de coeur pour l'utilisateur dans le signal BCG (12), les caractéristiques typiques debattement de coeur ayant été obtenue lors d'une étape d'apprentissage (10), **caractérisée en ce que** les caractéristiques typiques du battement cardiaque comprennent un vecteur caractéristique du modèle pour un battement cardiaque typique, $C_{HBcentre}$, et dans laquelle l'étape de détection des battements de coeur comprend:

- l'identification des points caractéristiques dans le signal BCG (203);
- la détermination des paramètres du signal BCG à chacun des points caractéristiques identifiés (204);
- la constitution d'une pluralité de vecteurs caractéristiques à partir des paramètres déterminés (205);
- et l'utilisation de la pluralité de vecteurs caractéristiques et $C_{HBcentre}$ pour détecter les battements cardiaques dans le signal BCG (206, 207, 208, 209, 210).

2. Une méthode selon la revendication 1, dans laquelle les caractéristiques typiques du battement de coeur comprennent en outre une partie d'un signal BCG utilisé dans l'étape d'apprentissage (10) correspondant au vecteur caractéristique du modèle, $S_{HBarch}$, et dans laquellle l'étape d'utilisation comprend:

- l'utilisation de la pluralité de vecteurs de caractéristiques, $C_{HBcentre}$ et $S_{HBarch}$ pour détecter les battements de coeur dans le signal BCG (206, 207, 208, 209, 210).

3. Une méthode selon la revendication 2, dans laquelle l'étape d'utilisation le calcul de la distance entre chacun des vecteurs de la pluralité de vecteurs caractéristiques et $C_{HBcentre}$ (206);

- l'identification des minima locaux dans les distances résultantes (206);
- la détermination de la corrélation croisée entre $S_{HBarch}$ et le signal BCG (207);
- l'identification des maxima locaux dans la corrélation croisée (207); et
- l'identification des battements de coeur dans le signal BCG) partir de l'emplacement des minima locaux dans les distances et des maxima locaux dans la corrélation croisée (208, 209, 210).

4. Une méthode telle que revendiquée dans la revendication 3, dans laquelle l'étape de détection des battements cardiaques (12) comprend en outre:

- le filtrage du signal BCG afin d'obtenir une enveloppe du composant de haute fréquence (208); et
- l'identification des maxima dans l'enveloppe du composant de haute fréquence (208);
- et dans laquelle l'étape consistant à identifier les battements cardiaques dans le signal BCG comprend:
- l'identification des battements cardiaques dans le signal de BCG à partir de l'emplacement des minima locaux dans les distances, des maxima locaux de la corrélation croisée et des maxima locaux dans l'enveloppe du composant de haute fréquence (209, 210).

5. Une méthode selon la revendication 4, dans laquelle l'étape d'identification des battements cardiaques dans le signal BCG comprend en outre:

- l'évaluation de la fiabilité de chacun des minima locaux dans les distances, des maxima locaux de la corrélation croisée et des maxima locaux dans l'enveloppe du composant de haute fréquence (209).

6. Une méthode selon la revendication 5, dans laquelle l'étape d'identification des battements cardiaques dans le signal BCG comprend en outre:

- la formation de triplets à partir des minima locaux dans les distances, des maxima locaux de la corrélation croisée et des maxima locaux dans l'enveloppe du composant de haute fréquence (210);
- la détermination d'une valeur représentative pour chaque triplet à partir de la fiabilité évaluée, la valeur représentative indiquant l'instant où a eu lieu un battement de coeur dans le signal BCG (210).

7. Une méthode selon une des revendications précédentes, la method comprenant en outre une étape d'apprentissage (10) dans laquelle des caractéristiques typiques du battement de coeur sont obtenues à partir d'une partie d'un signal BCG, $S_{train}$, l'étape d'apprentissage comprenant:

- l'identification des points caractéristiques dans $S_{train}$ (103);
- la détermination des paramètres de $S_{train}$ à chacun des points caractéristiques identifiés (104); la constitution d'une pluralité de vecteurs caractéristiques à partir des paramètres déterminés (105);
- le regroupement de la pluralité de vecteurs caractéristiques en une pluralité de groupes en fonction de la similitude des vecteurs caractéristiques (106);
- l'identification de groupes dans la pluralité de groupes liés à des battements de coeur (107), et
l'obtention de caractéristiques typiques du battement cardiaque en déterminant un vecteur caractéristique du modèle pour un battement de coeur typique, $C_{HBcentre}$, à partir des vecteurs caractéristiques dans le groupe et par la détermination d'une partie d'un signal BCG, $S_{HBarch}$, qui correspond au vecteur caractéristique du modèle (108, 109).

8. Une méthode selon la revendication 7, dans laquelle l'étape d'identification du groupe dans la pluralité de groupes liés à des battements de coeur (107) comprend:

pour chaque groupe dans la pluralité de groupes:

- la détermination d'un centre du groupe $f_{cm}$ (1071);
- l'identification du vecteur caractéristique $f_{archm}$ qui se rapproche le plus du centre du groupe $f_{cm}$ (1072);
- la localisation de la partie $s_{archm}$ du signal BCG qui correspond au vecteur caractéristique $f_{archm}$ (1073);
- le calcul de la fonction de distance entre le centre du groupe $f_{cm}$ et chaqu'un des vecteurs caractéristiques dans le groupe (1074);
- le calcul de la corrélation croisée entre $s_{archm}$ et le signal BCG (1075); et
- la détermination des estimations des emplacements des battements de coeur dans le signal BCG à partir de vecteurs caractéristiques dans le groupe avec un minimum local pour la fonction de distance et d'un maximum local de la corrélation croisée (1076);
- le filtrage du signal BCG $s_{train}$ pour identifier les emplacements des composants de haute fréquence (110), et
- l'identification du groupe lié au battement cardiaque comme étant le groupe qui a:

(i) les estimations des emplacements des battements de coeur qui sont espacées par des montants qui entrent dans un laps de temps spécifié (1077), et
(ii) les minima locaux dans la fonction de distance et les maxima locaux de la corrélation croisée qui coïncident le mieux avec l'emplacement des composants de haute fréquence (1078).

9. Une méthode selon une des revendications 1 à 8, dans laquelle les points caractéristiques dans le signal BCG comprennent les maxima dans le signal BCG.

10. Une méthode selon la revendication 9, dans laquelle les paramètres de chaque point maximum dans le signal BCG comprennent:

i) l'amplitude ($a_{max}$) du maximum,
ii) la distance ($d_{max}$) entre le maximum local et le minimum local suivant à droite,
iii) l'amplitude ($a_{min}$) du minimum local suivant à droite du maximum local, et
iv) la distance ($d_{min}$) entre le minimum local suivant vers la droite du maximum local, et le maximum local suivant à droite. $d_{min}$

11. Une méthode selon une des revendications 1 à 10, comprenant en outre l'étape consistant à:

- la réduction de la dimension des vecteurs caractéristiques en utilisant une analyse en composantes principales.

12. Une méthode selon une des revendications précédentes, comprenant en outre l'étape consistant à: affiner la localisation des battements cardiaques détectés dans le signal BCG, N battements cardiaques ayant été détecté à des instants $t_1$, $t_2$, ... , $t_n$ respectivement, en déterminant la valeur du paramètre dt qui maximise la corrélation croisée entre un segment du signal BCG entre deux battements cardiaques détectés à des instants tp et $t_{p+1}$ respectivement, où $1 \le p \le N-1$, et d'un segment ultérieur du signal BCG entre les instants ($t_{p+1}$ + dt) et l'instant ($2t_{p+1}$ - tp + dt).

13. Un appareil (304) pour une utilisation avec un dispositif (302) configuré pour mesurer u signal de ballistocardiogramme d'un utilisateur, l'appareil comprenant:

- des moyens (306) configurés pour recevoir un signal de ballistocardiogramme à partir du dispositif; et
- des moyens de traitement (308) configurés pour effectuer la méthode définie dans une des revendications 1 à 12, sur le signal de ballistocardiogramme reçu.

14. Un produit de programme informatique comprenant un code de programme informatique qui, lorsqu'il est exécuté sur un ordinateur ou un processeur, est configuré pour amener l'ordinateur ou le processeur à exécuter la méthode définie dans une des revendications 1 à 12.

```
┌─────────────────────────────┐
│   Analyse a BCG signal to   │
│  determine typical features │──── 10
│      of the heart beat      │
└─────────────────────────────┘
               │
               ▼
┌─────────────────────────────┐
│  Scan BCG signals for heart │
│ beats using typical features│──── 12
│    identified in step 10    │
└─────────────────────────────┘
```

# FIG. 1

# FIG. 2

100 — Obtain a training signal $s_{train}$ from ballistocardiograph

101 — Remove breathing component from signal by high pass filtering ($s_{train} \longrightarrow s_{filt}$)

110

Detect high frequency signal components using dynamically adjusted BP filter ($s_{filt} \longrightarrow s_{hfcomp}$)

102 — Smooth signal by low pass filtering ($s_{filt} \longrightarrow s_{smooth}$)

103 — Identify characteristic points in signal

104 — Parameterize $s_{smooth}$ at characteristic points

105 — Collect parameters of n characteristic points and use them to constitute feature vectors

106 — Cluster feature vectors by similarity

107 — Determine cluster $c_{HB}$ related with heart beats

108 — Determine cluster centre $c_{HBcenter}$ of $c_{HB}$

109 — Search for segment $s_{HBarch}$ in $s_{smooth}$ whose parameterization is closet to $c_{HBcenter}$

FIG. 3

FIG. 4

FIG. 5

FIG. 6

<table>
<tr><td>1071</td><td>Determine the cluster center $f_c$ for each of the M clusters</td></tr>
</table>

$$\downarrow$$

<table>
<tr><td>1072</td><td>Determine the feature vector $f_{archm} \varepsilon \{f_k | k=1,2, \ldots , K-n+1\}$ that is closest to each cluster centre</td></tr>
</table>

$$\downarrow$$

<table>
<tr><td>1073</td><td>Locate subsegment $s_{archm}$ of the training set $s_{smooth}$ that corresponds to $f_{archm}$</td></tr>
</table>

$$\downarrow$$

<table>
<tr><td>1074</td><td>Compute the distance function between $f_{cm}$ and the individual feature vectors $f_k$</td></tr>
</table>

$$\downarrow$$

<table>
<tr><td>1075</td><td>Compute the cross-correlation of $s_{archm}$ and $s_{smooth}$</td></tr>
</table>

$$\downarrow$$

<table>
<tr><td>1076</td><td>Determine estimates of heart beat locations from feature vectors with a local minimum for the distance function and a local maximum for the cross-correlation</td></tr>
</table>

$$\downarrow$$

<table>
<tr><td>1077</td><td>Exclude clusters that give estimates of heart beat locations that do not fall within a specified time window</td></tr>
</table>

$$\downarrow$$

<table>
<tr><td>1078</td><td>Identify cluster related to the heart beat from the respective local minima in the distance function, local maxima in the cross-correlation function and the location of high frequency components in $s_{hfcomp}$</td></tr>
</table>

# FIG. 7

FIG. 8

| 200 | Obtain a ballistocardiogram signal $s_{real}$ |
|---|---|

| 201 | Remove breathing component from signal by high pass filtering ($s_{real} \longrightarrow s_{filt}$) |
|---|---|

| 202 | Smooth signal by low pass filtering ($s_{filt} \longrightarrow s_{smooth}$) |
|---|---|

| 203 | Identify characteristic points in the signal $s_{smooth}$ |
|---|---|

| 204 | Parameterise $s_{smooth}$ at characteristic points |
|---|---|

| 205 | Collect parameters of n characteristic points and use them to constitute feature vectors |
|---|---|

| 206 | Compute the distance between the feature vectors and $c_{HBcenter}$ and identify local minima in the distance |
|---|---|

| 207 | Determine the cross-correlation between $s_{real}$ and $s_{HBcenter}$ and identify local maxima |
|---|---|

| 208 | Identify maxima in the high frequency envelope of $s_{filt}$ using the optimal bandpass filter from step 110 |
|---|---|

| 209 | Assess the reliability of the minima and maxima identified in steps 206, 207 and 208 |
|---|---|

| 210 | Determine the time a heart beat occurs from the minima and maxima identified in steps 206, 207 and 208 and their respective reliabilities |
|---|---|

# FIG. 9

FIG. 10

FIG. 11

FIG. 12

FIG. 13

FIG. 14

**EP 2 459 065 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- FFT averaging of multichannel BCG signals from bed mattress sensor to improve estimation of heart beat interval. **KORTELAINEN, J.M. ; VIRKKALA, J.** EMBS 2007, 29th Annual International Conference of the IEEE. Engineering in Medicine and Biology Society, 22 August 2007, 6685-6688
- Automatic Ballistocardiogram (BCG) Beat Detection Using a Template Matching Approach. **J.H. SHIN.** EMBS 2008, 30th Annual International Conference of the IEEE. Engineering in Medicine and Biology Society, 22 October 2008

- Estimation of Respiratory Waveform and Heart Rate Using an Accelerometer. **D.H. PHAN ; S. BONNET ; R. GUILLEMAUD ; E. CASTELLI, N.Y. ; PHAM THI.** 30th Annual International Conference of the IEEE. Engineering in Medicine and Biology Society, 21 August 2008
- **J. N. EDSON ; R. DICKES ; G. H. FLAMM ; M. TOBIN.** Higher Frequency Phenomena in the Normal Ballistocardiogram. *Circulation Research,* 1958, vol. 1, 405-409